(19) **Europäisches Patentamt / European Patent Office / Office européen des brevets**

(11) **EP 2 221 033 A1**

(12) 
# EUROPEAN PATENT APPLICATION
published in accordance with Art. 153(4) EPC

(43) Date of publication:
**25.08.2010 Bulletin 2010/34**

(21) Application number: **08854739.3**

(22) Date of filing: **18.08.2008**

(51) Int Cl.:
***A61F 13/49*** *(2006.01)*    ***A61F 13/514*** *(2006.01)*

(86) International application number:
**PCT/JP2008/064691**

(87) International publication number:
**WO 2009/069343 (04.06.2009 Gazette 2009/23)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **28.11.2007 JP 2007307998**

(71) Applicant: **Unicharm Corporation
Ehime 799-0111 (JP)**

(72) Inventors:
• **MUKAI, Hirotomo**
  **Kanonji-shi**
  **Kagawa 769-1602 (JP)**
• **TSUJI, Tomoko**
  **Kanonji-shi**
  **Kagawa 769-1602 (JP)**
• **HASHIMOTO, Tatsuya**
  **Kanonji-shi**
  **Kagawa 769-1602 (JP)**
• **WATABE, Yoshihisa**
  **Kanonji-shi**
  **Kagawa 769-1602 (JP)**

(74) Representative: **Eke, Philippa Dianne
Saunders & Dolleymore LLP
9 Rickmansworth Road
Watford
Hertfordshire WD18 0JU (GB)**

(54) **ABSORBENT ARTICLE TO BE WORN**

(57)     The present invention aims to provide an absorbent wearing article having a feeling to wear well improved.

An absorbent wearing article 1 comprises an absorbent structure 8 and a cover sheet 7 defining an outermost layer wherein the cover sheet 7 is formed from a fibrous nonwoven fabric containing moisture-absorbent fibers and hydrophobic fibers.

FIG.1

EP 2 221 033 A1

**Description**

TECHNICAL FIELD

The present invention relates to an absorbent article and more particularly to an absorbent wearing article such as disposable diapers, toilet training pants, incontinence pants or sanitary napkins, each having improved a feeling to wear.

RELATED ART

For example, JP 2007-97979A discloses a disposable absorbent article comprising a chassis sheet having a rear waist region, a front waist region and a crotch region extending between these front and rear waist regions and an absorbent structure extending across the crotch region further into the front and rear waist regions, wherein an outer layer sheet defining an outermost layer of the chassis sheet is formed by a nonwoven fabric sheet having been heat-embossed and modified to become hydrophilic and has an average friction coefficient in a range of 0.130 to 0.150.
PATENT DOCUMENT 1: JP 2007-97979A

DISCLOSURE OF THE INVENTION

PROBLEM TO BE SOLVED BY THE INVENTION

According to the disclosure of JP 2007-97979A, a feeling of roughness as well as a feeling of friction is effectively alleviated and thereby the absorbent article having a smooth and soft touch just like undergarments.
However, when the fibrous nonwoven fabric sheet having been modified to become hydrophilic is used as the outer sheet of the diaper, bodily fluids may leach out from the absorbent structure through the outer layer to the exterior of the diaper and soil the wearer's garment such as trousers unless the inner surface of the outer layer is provided with a liquid-impervious sheet.
The present invention provides an absorbent wearing article being able to prevent bodily fluids from leaching through the article to the exterior thereof and to offer a comfortable feeling to wear.

MEASURE TO SOLVE THE PROBLEM

According to the present invention, there is provided an absorbent wearing article comprising an absorbent structure and a cover sheet defining an outermost layer when the article is put on the wearer's body. The present invention is **characterized in that** the cover sheet is formed from a fibrous nonwoven fabric containing moisture-absorbent fibers and hydrophobic fibers.
The present invention may be implemented in preferred embodiments as will be described below.
According to one preferred embodiment, the fibrous nonwoven fabric forming the cover sheet has a moisture absorption coefficient in a range of 0.10 to 0.27% calculated by a formula 1:

[Formula 1]

$$\text{Moisture absorption coefficient} = \frac{(b - c)}{c} \times 100 (\%) \qquad \text{(Formula 1)}$$

on the basis of mass b (g) of a test piece determined after the test piece has been left within a chamber at constant temperature and humidity (20°C, 60%RH) for 24 hours and then subjected to moisture absorption for 2 hours within a chamber at constant temperature and humidity (40°C, 60%RH) and mass c (g) of the test piece determined after the test piece had been subjected to moisture absorption has been dried within an oven at a temperature of 105°C ± 3°C.
According to another preferred embodiment, the cover sheet comprises at least a first layer of hydrophobic fibers having been modified to become hydrophilic and a second layer of hydrophobic fibers having not been modified to become hydrophilic and wherein the first layer defines an outermost surface of the cover sheet.
According to still another preferred embodiment, the hydrophobic fibers having been modified to become hydrophilic are heat fused with the hydrophobic fibers having not been subjected to such modification in the cover sheet.
According to yet another preferred embodiment, the hydrophobic fibers having been modified to become hydrophilic are hydrophobic fibers containing the hydrophilic property imparting modifier kneaded therein.

According to further another preferred embodiment, the cover sheet comprises a front waist region having a front waist band region defining a front waist upper end, a rear waist region having a rear waist band region defining a rear waist upper end, a crotch region extending between the front and rear waist regions and a liquid-impervious barrier sheet provided at least in the crotch region;

the absorbent structure is provided at least in the crotch region; and

the liquid-impervious barrier sheet is interposed between the absorbent structure and the cover sheet.

According to an alternative preferred embodiment, the cover sheet is provided at least in the front waist band region and/or the rear waist band region.

The term "hydrophobic property" used herein refers to the artificial urine permeability of the stipulated value (ten points or spots) or less.

EFFECT OF THE INVENTION

The absorbent wearing article according to the present invention has its outermost surface defined by the cover sheet containing moisture-absorbent fibers adapted to improve a touch peculiar to the fibrous nonwoven fabric. As a consequence, a feeling of softness is experienced by the wearer when he or she touches the article and a feeling to wear is also correspondingly improved. The cover sheet contains hydrophobic fibers adapted to assure a sufficiently high degree of hydrophobic property and liquid-imperviousness to prevent bodily fluids from oozing out through the absorbent wearing article even if the barrier sheet is defined by the cover sheet alone.

The moiety of component fibers having been modified to become hydrophilic in the cover sheet absorbs an amount of moisture in ambient air or leaking from the absorbent wearing article itself and thereby maintains an appropriate degree of humidity.

The cover sheet assures a comfortable feeling to wear the absorbent wearing article so far as the moisture absorption coefficient defined by said formula 1 is in the range of 0.10 to 0.27%.

According to the embodiment wherein the cover sheet comprises a layer of hydrophobic fibers having been modified to become hydrophilic and a layer of hydrophobic fibers having not been modified to become hydrophilic and the layer having been modified to become hydrophilic defines the outermost surface of the cover sheet, the layer having been modified to become hydrophilic assures a reasonable degree of moist feel and a comfortable soft touch experienced by the wearer. The layer of hydrophobic fibers having not been subjected to such modification prevents bodily fluids from leaking out through the absorbent wearing article. In this way, a feeling to wear the absorbent wearing article is effectively improved.

According to the embodiment wherein the hydrophobic fibers having been modified to become hydrophilic are heat fused with the hydrophobic fibers having not been subjected to such modification in the cover sheet, a desired air-permeability of the nonwoven fabric should not be deteriorated as bonding by means of hot melt adhesive (referred to hereinafter simply as HMA) has been the case. In addition, the method of heat fusing these two layers ensures a sufficiently high tensile strength of the absorbent wearing article to prevent the article from unintentionally broken and, in consequence, the wearer can use the article at ease.

Kneading the hydrophilic property imparting modifier in the hydrophobic fibers makes it possible to avoid a problem that the hydrophilic property imparting modifier might be removed from the fibers due to friction and/or evaporation and, as a result, the desired effect of the modifier might be reduced.

BRIEF DESCRIPTION OF THE DRAWINGS

[FIG. 1] Fig. 1 is a perspective view of a diaper.
[FIG. 2] Fig. 2 is a plan view of the flatly developed diaper.
[FIG. 3] Fig. 3 is a sectional view taken along the line III-III in Fig. 2.
[FIG. 4] Fig. 4 is a diagram schematically illustrating texture of a cover sheets.
[FIG. 5] Fig. 5 is a diagram schematically illustrating texture of the cover sheets.

IDENTIFICATION OF REFERENCE NUMERALS USED IN THE DRAWINGS

1    diaper (absorbent wearing article)
2    front waist region

| 3 | crotch region |
|---|---|
| 4 | rear waist region |
| 5 | topsheet |
| 6 | barrier sheet |
| 7 | cover sheets |
| 8 | absorbent structure |
| 17 | front waist band region |
| 17a | front waist region's end |
| 18 | rear waist band region |
| 18a | rear waist region's end |
| 22 | moisture-absorbent fiber |
| 23 | hydrophobic fiber |
| 24 | first layer |
| 25 | second layer |

IDENTIFICATION OF REFERENCE NUMERALS USED IN THE DRAWINGS

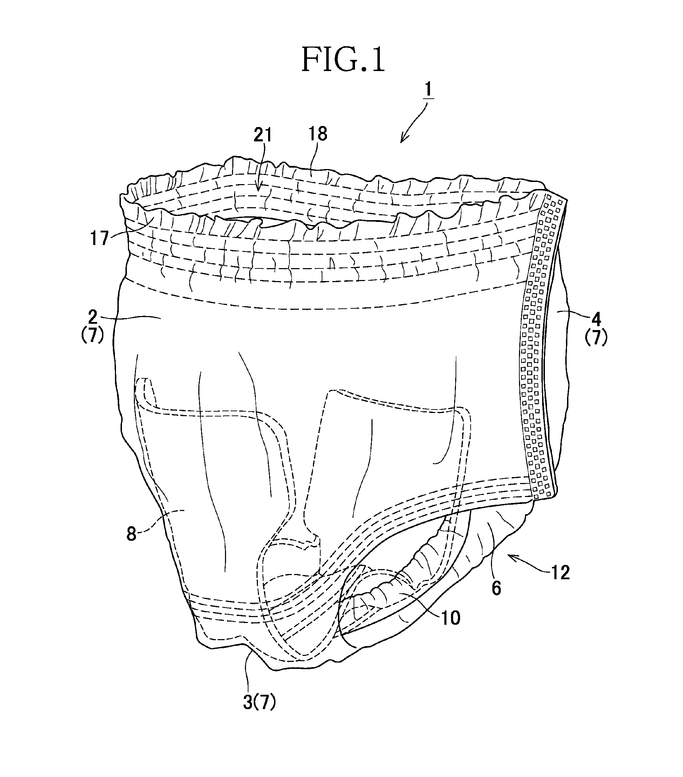

Details of the present invention will be more fully understood from the description of the absorbent wearing article implemented in the form of a disposable diaper 1 (referred to hereinafter simply as diaper 1). As shown in Fig. 1 in the perspective view, the diaper 1 comprises a front waist region 2 adapted to cover the wearer's ventral side, a rear waist region 4 adapted to cover the wearer's dorsal side and a crotch region 3 adapted to cover the wearer's crotch region. The front waist region 2, the rear waist region 4 and the crotch region 3 are fully covered with a backsheet of the diaper 1, i.e., a cover sheet 7 defining the outermost layer of the diaper 1. In addition, the diaper 1 is provided on its inner side with an absorbent structure 8 and barrier cuffs 10.

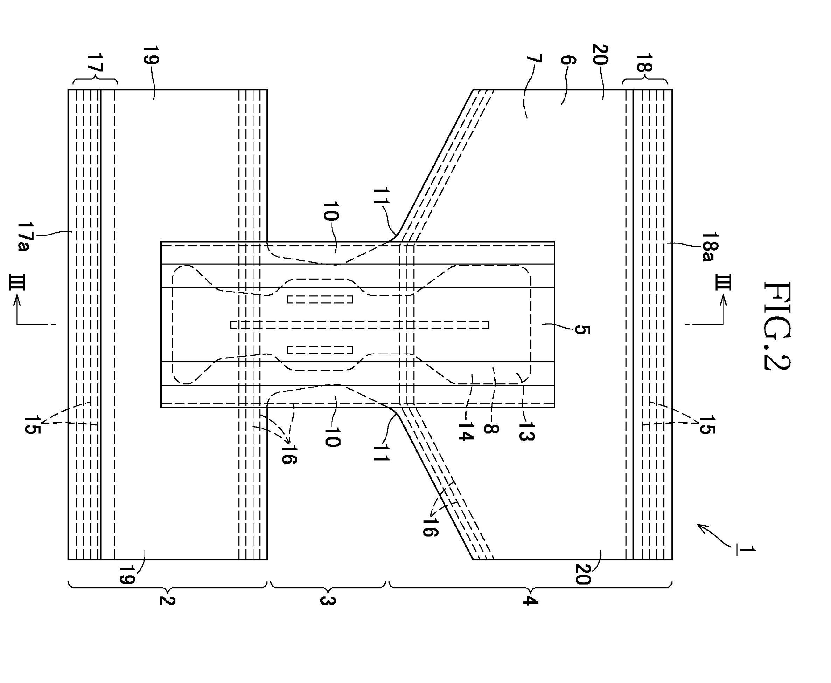

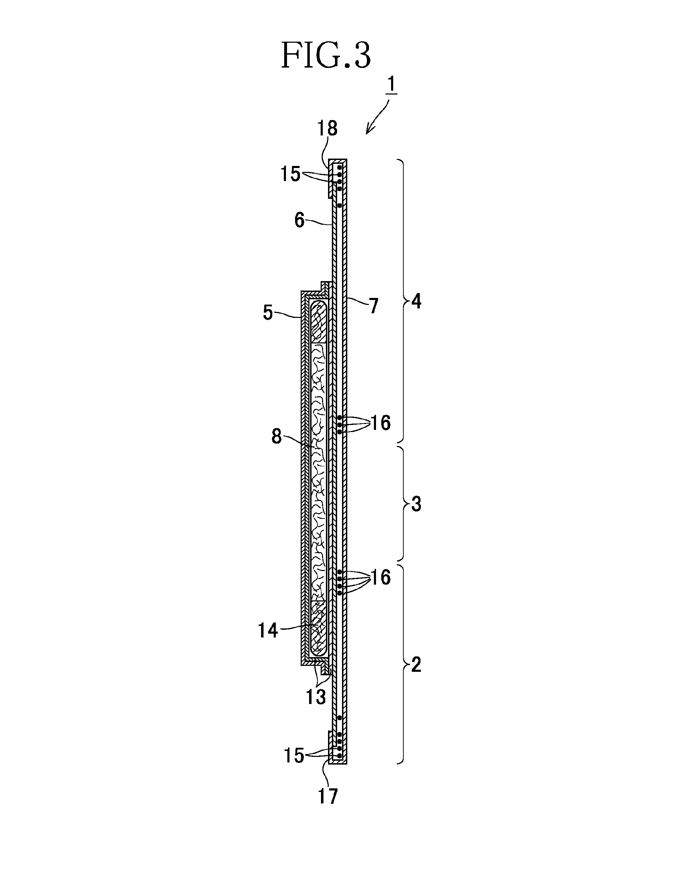

Fig. 2 shows the diaper 1 as has been flatly developed and viewed from the inner side (i.e., the side opposed to the cover sheets 7) and Fig. 3 is a sectional view taken along the line III-III in Fig. 2. Referring to Figs. 2 and 3, the diaper 1 comprises a topsheet 5 facing the wearer's skin when the diaper 1 is put on the wearer's body, a barrier sheet 6 lying on the opposite side of the topsheet 5 and the cover sheets 7 lying on the outer side of the barrier sheet 6. As has previously been described, the diaper 1 comprises the front waist region 2, the rear waist region 4 and the crotch region 3 extending between and being contiguous to these front and rear waist regions 2, 4.

In the diaper 1, transversely opposite side edges 11, 11 of the crotch region 3 are concavely curved inwardly of the diaper 1 and define a pair of leg-openings 12 as the diaper 1 is shaped in a pant. Transversely opposite side edges 19 of the front waist region 2 including a front waist band region 17 which defines, in turn, a front waist region's end 17a are respectively bonded to transversely opposite side edges 20 of the rear waist region 4 including a rear waist band region 18 which defines, in turn, a rear waist region's end 18a to shape the diaper 1 in pull-on pants whereupon a waist-opening 21 is formed. Instead of bonding the front and rear waist regions 2, 4 along the side edges thereof in this manner, it is also possible to attach tape fasteners to the side edges 20 of the rear waist region 4 so that these fasteners may be detachably jointed to the outer surface of the front waist region 2 to obtain a so-called open-type diaper.

The absorbent structure 8 may be of the well known structure in which an absorbent core 14 is interposed between upper and lower wrap sheet layers 13. The absorbent structure 8 is provided so as to extend across the crotch region 3 and further into the front waist region 2 and the rear waist region 4 wherein the absorbent structure 8 is interposed and bonded between the topsheet 5 and the barrier sheet 6 by means of hot melt adhesive (referred hereinafter to as HMA and not shown). The diaper 1 further comprises the components conventionally used in the relevant field of art, for example, a pair of the barrier cuffs 10, waist elastic members 15 attached under tension to the front and waist regions along the ends 17a, 18a thereof and leg elastic members 16 attached under tension to the crotch region 3 along the transversely opposite side edges 11, 11 thereof.

The topsheet 5 is formed from a liquid-pervious fibrous nonwoven fabric or plastic film (e.g., porous plastic film) and the wrap sheet 13 is formed from a liquid-pervious fibrous nonwoven fabric or a plastic film (e.g., porous plastic film) or a tissue paper. As will be seen in Fig. 3, the topsheet 5 is bonded to the wrap sheet 13 by means of HMA and lies on the innermost side of the diaper 1 so as to face the wearer's crotch region in the diaper 1 being in a pant-shape. Bodily fluids discharged permeate the topsheet 5 and the wrap sheet 13 in this order and are absorbed by the absorbent core 14.

The barrier sheet 6 is formed from an air-permeable and liquid-impervious plastic film or a fibrous nonwoven fabric and bonded to the bottom surface of the absorbent structure 8 by means of HMA. The barrier sheet 6 bonded to the bottom surface of the absorbent structure 8 extends outward beyond a peripheral edge of the absorbent structure 8 in the longitudinal direction as well as in the transverse direction. While not illustrated, it is also possible to interpose a separately prepared liquid-impervious plastic film between the absorbent structure 8 and the barrier sheet 6 or between the barrier sheet 6 and the cover sheets 7 defining the backsheet. The barrier sheet 6 covers a substantially entire area of the front and rear waist regions 2, 4 and the crotch region 3 in order to prevent any amount of bodily fluids once absorbed by the absorbent core 14 from leaking outwardly of the diaper 1. It should be understood that the barrier sheet 6 may effectively function so far as the barrier sheet 6 at least underlies the absorbent structure 8.

As for the quality of material, the barrier sheet 6 may be appropriately varied depending on factors such as a particular purpose of utilization. For example, the material for the barrier sheet 6 may be selected from the group consisting of a spun bond nonwoven fabric, a spun bond-melt blow-spun bond (SMS) nonwoven fabric, a point bond nonwoven fabric, a through-air nonwoven fabric, a spun lace nonwoven fabric, a needle punch nonwoven fabric and the other types of fibrous nonwoven fabric. These various types of nonwoven fabric may be used individually or in combination. As component fiber constituting each of these nonwoven fabrics, polyolefin-based, polyamide-based fibers or polyethylene/ polypropylene or polyethylene/polyester core-sheath type composite fibers or side-by-side type composite fibers is useful. Mass of the component fiber is preferably in a range of 12 to 30 g/m$^2$ and an apparent density of the component fibers is preferably in a range of 0.02 to 0.2 g/cm$^3$.

The cover sheets 7 are formed from a fibrous nonwoven fabric containing moisture-absorbent fibers and hydrophobic fibers so that the cover sheets 7 are air-permeable but liquid-impervious. The cover sheets 7 are bonded to the outer surface of the barrier sheet 6 by means of HMA and entirely cover the front and rear waist regions 2, 4 and the crotch region 3 of the diaper 1. In other words, the cover sheets 7 cover a region surrounding the wearer's waist, a region in which the absorbent structure 8 is present and the remaining region. In this manner, the cover sheets 7 define the outer sheet of the diaper 1. The hydrophobic fibers contained in the cover sheets 7 makes the cover sheets 7 as a whole hydrophobic. The hydrophobic property of the cover sheets 7 preferably assure a sufficient liquid-resistance or a barrier effect to prevent bodily fluids from permeating the cover sheets 7 even when said barrier sheet 6 is not used. However, the liquid-resistance or barrier effect of such high degree as has been described above will not necessarily be required so far as a comfortable feeling and a soft touch can be provided by the cover sheets 7 when the barrier sheet 6 is used.

The cover sheets 7 may be formed from a process for making a nonwoven fabric such as a carding process, a spun bond process, a melt blow process or the other process. For example, a spun bond nonwoven fabric made of polypropylene-based thermoplastic resin is preferable. To maintain a strength required for the outer sheet of the diaper 1 and at the same time to obtain a soft touch, an appropriate strength is required in the longitudinal direction as well as in the transverse direction. In view of this, a spun bond nonwoven fabric or an SMS nonwoven fabric presenting a well balanced longitudinal and transverse strength is suitable.

The cover sheets 7 are respectively folded back along the front waist region's upper end 17a and the rear waist region's upper end 18a onto the surface of the barrier sheet 6 on which the absorbent structure 8 is attached, i.e., onto the inner side of the diaper 1 and placed on and bonded to the barrier sheet 6. Ranges in which the cover sheets 7 overlaps the barrier sheet 6 are preferably in the regions of the barrier sheet 6 in which the waist elastic members 15 are present. With such an arrangement, the moisture-absorbent cover sheets 7 come in contact with the wearer's skin along the front waist region's upper end 17a and the rear waist region's upper end 18a when the diaper 1 has been put on the wearer's body and this feeling to wear the diaper 1 is improved. When the diaper 1 is pulled up, the hands of the wearer or the helper come in contact with the inner side and the outer side of the diaper 1. The cover sheets 7 may be folded back onto the inner side of the diaper 1 to ensure that the hands reliably come in contact with the cover sheets 7 when the diaper 1 is pulled up. In consequence, a desired soft touch of the diaper 1 is further improved.

According to the present invention, the cover sheets 7 may be provided at least in the front waist band region 17 and/or the rear waist band region 18. While not illustrated, this requirement may be met also by preparing a pair of cover sheets each having the size corresponding to the length and the width of each of the band regions 17, 18 and bonding them to the inner surface and/or the outer surface of the respective band regions 17, 18.

When the cover sheets 7 are bonded to the barrier sheet 6 by means of HMA, these sheets 7, 6 may be intermittently coated with HMA to ensure a desired air-permeability between these sheets. More specifically, the sheets may be intermittently coated with HMA in a dot pattern, a spiral pattern or a stripe pattern.

The air-permeable fibrous nonwoven fabric or the like is used to form the barrier sheet 6 so that the wearer might not experience a feeling of stuffiness. However, water vapor may be rarely condensed to form drops of water on the surface of the barrier sheet 6 as if the sheet 6 exudes perspiration and, in this case, the wearer may experience an uncomfortable feeling. When the barrier sheet 6 and the cover sheets 7 are bonded together in the manner as has been described above, drops of water having been accumulated between the barrier sheet 6 and the cover sheets 7 gradually evaporate from the cover sheets 7. Thus the wearer should not experience an uncomfortable feeling and, in consequence, the diaper 1 offering a good feeling to wear is obtained.

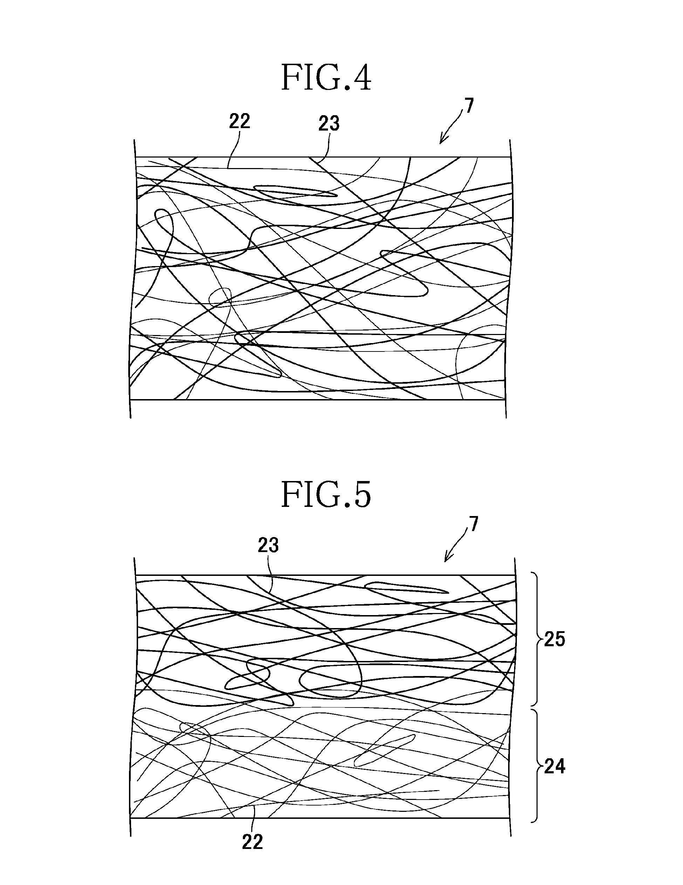

As the moisture-absorbent fibers constituting the cover sheets 7, hydrophobic synthetic fibers modified to become hydrophilic, regenerated or semi-synthetic fibers such as rayon or moisture-absorbent natural fibers such as cotton may be used. Obviously, the hydrophobic fibers may be used without being modified to become hydrophilic. As schematically illustrated in Fig. 4, the cover sheets 7 may be implemented also in the form of commingled fibers of moisture-absorbent fibers 22 indicated by thin lines and hydrophobic fibers 23 indicated by heavy lines. In the case of such commingled fibers, the cover sheets 7 as a whole preferably have mass in a range of 12 to 30 g/m$^2$ and a density

in a range of 0.02 to 0.2 g/cm$^3$, and the moisture-absorbent fibers 22 preferably have percent by mass in a range of 20 to 80 and a fineness in a range of 10 to 35 $\mu$m. The fibers 23 preferably have a fineness in a range of 10 to 35 $\mu$m.

As schematically illustrated in Fig. 5, each of the cover sheets 7 may be implemented in the form of a laminate consisting of a first layer 24 made of moisture-absorbent fibers 22 and a second layer 25 made of hydrophobic fibers 23. While it is possible to bond these first and second layers 24, 25 to each other by means of HMA intermittently applied between these layers, the first and second layers 24, 25 are maintained in a laminated state preferably by interlacing or heat-sealing the component fibers together.

In this regard, it is also possible to implement the cover sheet 7 in the form of some other variants, while not illustrated herein, for example, a laminate in which the first layer 24 of commingled fibers principally composed of hydrophobic fibers, a laminate in which the second layer 25 of hydrophobic fibers are partially scattered within the first layer 24 of moisture-absorbent fibers 22, or a multilayered structure such as a laminate in which the hydrophobic second layer is interposed between a pair of the moisture-absorbent first layers 24.

Both the hydrophobic fibers having been modified to become hydrophilic and the hydrophobic fibers having not been subjected to such modification may be produced from the same or different type of thermoplastic resins. It is also possible to obtain composite fibers of core/sheath type from two different types of raw resin.

To modify the hydrophobic fibers to become hydrophilic, nonionic or anionic surfactant of well known art may be used as hydrophilic property imparting modifiers for fibers. Such modification is achieved by attaching or kneading the hydrophilic property imparting modifier to or into the hydrophobic fibers. To avoid a possibility that the hydrophilic property imparting modifier might be separated from the fibers due to friction or evaporation, it is preferred to knead the hydrophilic property imparting modifier into the hydrophobic fibers.

For example, the hydrophobic fibers may be modified by kneading therein a hydrophilic property imparting modifier having a chemical formula:

$$[\text{Chemical Formula 1}] \qquad R_1\text{-(hydrophilic oligomer)}$$

(wherein $R_1$ represents a straight chain alkyl group or a branched chain alkyl group and a hydrophilic oligomer represents homo-oligomer or co-oligomer of 2 to 10 monomer units derived from a monomer selected from the group consisting of ethylene oxide, propylene oxide, ethylene glycol, propylene glycol, epichlorohydrin, acrylic acid, methacrylic acid, ethylene-imine, caprolactam, vinyl alcohol and vinyl acetate.)

As has previously been described, the cover sheet 7 may be implemented in the form of the commingled fibers composed of moisture-absorbent fiber and hydrophobic fibers or the laminate of the moisture-absorbent fibers layer and the hydrophobic fiber layer. Such laminate can be obtained, for example, by forming a nonwoven fabric from hydrophobic fibers modified to become hydrophilic by the spun bond process and immediately followed by laminating a nonwoven fabric of hydrophobic fibers subjected not to modification treatment on the former nonwoven fabric through the spun bond process or by lamination in the reverse order.

In the case of the cover sheet 7 laminated in the manner as has been described just above, the hydrophobic fibers having been modified to become hydrophilic and the hydrophobic fibers having not been subjected to such modification are mixed one with another and heat fused together at many points between those two layers. Consequentially, the air-permeability of the laminate should not be deteriorated as the two layers bonded to each other is the case and a comfortable feeling to wear can be assured when this laminate is used in the absorbent article. In addition, a plurality of heat fusion points formed among the fibers ensures a desired tensile strength and, for example, when such cover sheet is used for the diaper 1, the boundary between those two layers or the laminate (i.e., the cover sheet 7) itself should not be unintentionally broken as the diaper 1 is pulled up with the hands. In view of this advantageous effect, this laminate is suitable as the cover sheet 7 used in the front and rear regions 2, 4 as well as in the crotch region 3 containing the absorbent structure 8.

The cover sheet 7 comprises the hydrophobic fibers provided with the hydrophilic property imparting modifier kneaded therein exhibits an appropriate degree of moisture-absorbent capacity so that the wearer may experience a reasonable degree of moist feeling when the wearer's hands come in contact with the region of the diaper 1 in which the cover sheet 7 is present. Thus, a feeling of dryness and/or a feeling of roughness as if the wearer's hands are caught by the fibers of the diaper 1, which would otherwise be experienced by the wearer when the wearer tries to pull the diaper 1 up can be effectively alleviated. Now evaluation of feel of the diaper 1 against the wearer's skin will be discussed.

Based on test pieces of the cover sheet having different degrees of moisture-absorption coefficient, a feel of the diaper 1 against the wearer's skin such as a feel of moistness and a feel of softness and hydrophilic/hydrophobic properties were evaluated and a result as indicated in TABLE 1 was obtained.

For the evaluation, a polypropylene (PP) spun bond nonwoven fabric of polypropylene (PP) used in the conventional diaper was used as the comparative example 1.

Test pieces Nos. 1 - 3 were also a PP spun bond nonwoven fabric similar to the comparative example 1 except that each of these test pieces consists of two layers having the substantially same thickness, one of which contains hydrophilic property imparting modifier kneaded therein and the other containing not such modifier. The amount of the hydrophilic property imparting modifier to be kneaded into the PP fibers was varied to adjust the moisture absorption coefficient of the nonwoven fabric. The additive rate (mass %) of the hydrophilic property imparting modifier was gradually increased in the order of the sample number, i.e., in the order of the samples 1 to the sample 3.

As the comparative example 2, a single layer of spun bond PP nonwoven fabric containing the hydrophilic property imparting modifier kneaded therein at the same additive rate (mass %) as in the case of the test piece No. 3 was used.

The layer constituting fibers of the test pieces Nos. 1 - 3 and the comparative examples 1, 2 respective had a mass of 17 g/m$^2$, a density of 0.085 g/cm$^3$ and a fineness of 18.2 $\mu$m.

The additive rate of the hydrophilic property imparting modifier for hydrophobic fibers such as PP is usually in a range of 0.1 mass % to 3.0 mass %. If it exceeds 3.0 mass %, the absorbent article added with this should deteriorate the leak-proof effect.

Referring to TABLE 1, the moisture absorption coefficient was calculated by said formula 1 on the basis of mass b (g) of the test piece determined after the test piece has been left within a chamber at constant temperature and humidity (20°C, 60%RH) for 24 hours and then subjected to moisture absorption for 2 hours within a chamber at constant temperature and humidity (40°C, 60%RH) and mass c (g) of the same test piece determined after this test piece had been subjected to moisture absorption has been dried within an oven at a temperature of 105°C $\pm$ 3°C. Evaluation of hydrophobic property/hydrophilic property was carried out by the following steps as will be described.

(1) A stack of five sheets of filter paper was kept horizontal and the test piece was placed thereon.
(2) One drop (about 0.05 g) of artificial urine was dropped onto the test piece at twenty points thereon through a pipette.
(3) 30 seconds after the artificial urine had been dropped onto the test piece at twenty points, the number of points at which the artificial urine had been absorbed by the sheets of filter paper was counted (inclusive of the number of points at which the artificial urine had not been absorbed by the sheets of filter paper but had sunk in the test piece).
(4) The test piece was determined as a hydrophobic sheet when the number of points at which the artificial urine had been absorbed by the sheets of filter paper through the test piece had been counted to be 0 to 10 and was determined as a hydrophilic sheet when the number has been counted to be 11 to 20.

The sensory evaluation was carried out by twenty persons (N = 20) on the layer in which the hydrophilic property imparting modifier had been kneaded.

[TABLE 1]

| Test piece Nos. | Comp. Ex. 1 | Example 1 | Example 2 | Example 3 | Comp. Ex. 2 |
|---|---|---|---|---|---|
| Nonwoven fabric's type | Hydro-phobic | < amount of H-philic property imparting modifier < | | | |
| Moisture Absorption (%) | 0 | 0.105 | 0.212 | 0.264 | - |
| H-phobic/ H-philic | | Hydro-phobic | Hydro-phobic | Hydro-phobic | Hydro-philic |
| Has it moist feel? | No | Yes | Yes | Yes | Yes |
| Softness Score | A | A - B | B | C | - |

1) A: moderate (Comparative Ex.)

B: softer than Comparative Ex.

C: substantially softer than Comparative Ex.

Compared to the comparative examples, the moisture-absorbent test pieces 1 - 3 respectively exhibited a reasonable degree of moist feel on the surfaces thereof and the softness score increased substantially in proportion to the absorption coefficient.

On the comparative example 2 consisting of the single layer containing the hydrophilic property imparting modifier, it was found from evaluation of hydrophobic property/hydrophilic property that an amount of the artificial urine absorbed by the sheets of filter paper through said single layer is not negligible and the leak-proof effect is correspondingly poor.

With the view of the result of evaluation as has been described above, the nonwoven fabric according to the present invention which consists of the layer containing the hydrophilic property imparting modifier kneaded therein and the layer containing not such modifier is suitable as the cover sheet 7 in the absorbent article particularly when the it is incorporated in the article so that the layer containing the modifier kneaded therein faces away from the wearer's skin. This is for the reason that the region of the absorbent article apt to come in contact with the hands of the wearer him- or herself or the helper may assure an appropriate degree of moist feel and thereby alleviate an uncomfortable feeling of dryness and/or a feeling of roughness as if the hands of the wearer or the helper are caught by the fibers. In this way, a feeling of softness of the article against the wearer's skin can be improved.

The cover sheet 7 includes a moiety of unmodified hydrophobic fibers contributing to prevent bodily fluids from exuding to the outer surface of the cover sheet and eventually leaking out to the outer side of the absorbent article. In addition, the cover sheet 7 facilitates the aged wearer often suffering from so-called dry skin to handle the article.

Furthermore, relatively high moisture absorption coefficient assured by the cover sheet advantageously restrains generation of static electricity particularly in a winter season and facilitate the material to be handled particularly in a winter season. Also during use of the article, such high moisture absorption coefficient advantageously restrains troubles due to generation of static electricity.

It is also possible to implement the present invention in a manner that the cover sheets 7 with the layer thereof containing the hydrophilic property imparting modifier kneaded therein facing the wearer's skin serve as the barrier cuffs 10. In addition, the cover sheet 7 may be used as sanitary napkins or component nonwoven fabrics thereof or packaging sheet for such articles.

**Claims**

1. An absorbent wearing article comprising an absorbent structure and a cover sheet defining an outermost layer when the article is put on the wearer's body, said absorbent wearing article being **characterized in that**:

   said cover sheet is formed from a fibrous nonwoven fabric containing moisture-absorbent fibers and hydrophobic fibers.

2. The absorbent wearing article as recited by Claim 1, wherein said fibrous nonwoven fabric forming said cover sheet has a moisture absorption coefficient in a range of 0.10 to 0.27% calculated by a formula 1:

   [Formula 1]

   $$\text{Moisture absorption coefficient} = \frac{(b - c)}{c} \times 100 (\%) \qquad \text{(Formula 1)}$$

   on the basis of mass b (g) of a test piece determined after said test piece has been left within a chamber at constant temperature and humidity (20°C, 60%RH) for 24 hours and then subjected to moisture absorption for 2 hours within a chamber at constant temperature and humidity (40°C, 60%RH) and mass c (g) of said test piece determined after said test piece had been subjected to moisture absorption has been dried within an oven at a temperature of 105°C $\pm$ 3°C.

3. The absorbent wearing article as recited by Claim 1 or 2, wherein said cover sheet comprises at least a first layer of hydrophobic fibers having been modified to become hydrophilic and a second layer of hydrophobic fibers having not been modified to become hydrophilic and wherein said first layer defines an outermost surface of said cover sheet.

4. The absorbent wearing article as recited by Claim 3, wherein said hydrophobic fibers having been modified to become hydrophilic are heat fused with said hydrophobic fibers having not been subjected to such modification in said cover sheet.

5. The absorbent wearing article as recited by Claim 4, wherein said hydrophobic fibers having been modified to become hydrophilic are hydrophobic fibers containing the hydrophilic property imparting modifier kneaded therein.

6. The absorbent wearing article as recited by any one of Claims 1 through 5, wherein:

   said cover sheet comprises a front waist region having a front waist band region defining a front waist upper end, a rear waist region having a rear waist band region defining a rear waist upper end, a crotch region extending between said front and rear waist regions and a liquid-impervious barrier sheet provided at least in said crotch region;

said absorbent structure is provided at least in said crotch region; and

said liquid-impervious barrier sheet is interposed between said absorbent structure and said cover sheet.

7.   The absorbent wearing article as recited by Claim 6, wherein said cover sheet is provided at least in said front waist band region and/or said rear waist band region.

# FIG.1

# FIG.2

# FIG.3

## FIG.4

## FIG.5

**INTERNATIONAL SEARCH REPORT**

| | International application No. |
|---|---|
| | PCT/JP2008/064691 |

A.   CLASSIFICATION OF SUBJECT MATTER
*A61F13/49(2006.01)i, A61F13/514(2006.01)i*

According to International Patent Classification (IPC) or to both national classification and IPC

B.   FIELDS SEARCHED

Minimum documentation searched (classification system followed by classification symbols)
A61F13/49, A61F13/514

Documentation searched other than minimum documentation to the extent that such documents are included in the fields searched
Jitsuyo Shinan Koho           1922-1996   Jitsuyo Shinan Toroku Koho   1996-2008
Kokai Jitsuyo Shinan Koho   1971-2008   Toroku Jitsuyo Shinan Koho   1994-2008

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| X<br>A | JP 11-216163 A  (Oji Paper Co., Ltd.),<br>10 August, 1999 (10.08.99),<br>Claim 1; Par. No. [0009]; Figs. 1 to 3<br>Claim 1; Par. No. [0009]; Figs. 1 to 3<br>(Family: none) | 1,6-7<br>2-5 |
| X<br>A | JP 2003-153939 A  (Livedo Corp.),<br>27 May, 2003 (27.05.03),<br>Par. Nos. [0010], [0016]<br>Par. Nos. [0010], [0016]<br>(Family: none) | 1,6-7<br>2-5 |
| X<br>A | JP 2002-272787 A  (Livedo Corp.),<br>24 September, 2002 (24.09.02),<br>Par. No. [0019]<br>Par. No. [0019]<br>(Family: none) | 1,6-7<br>2-5 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 November, 2008 (11.11.08) | 25 November, 2008 (25.11.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2008/064691

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2007-97979 A  (Livedo Corp.),<br>19 April, 2007 (19.04.07),<br>Par. Nos. [0008], [0018], [0023], [0026];<br>Figs. 1 to 4<br>(Family: none) | 1-7 |
| Y | JP 2001-198997 A  (Uni-Charm Corp.),<br>24 July, 2001 (24.07.01),<br>Par. Nos. [0001], [0005], [0017], [0026];<br>Figs. 2 to 3<br>(Family: none) | 1-7 |

Form PCT/ISA/210 (continuation of second sheet) (April 2007)

## INTERNATIONAL SEARCH REPORT

| | |
|---|---|
| | International application No. |
| | PCT/JP2008/064691 |

| **Box No. II** | **Observations where certain claims were found unsearchable (Continuation of item 2 of first sheet)** |
|---|---|

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| **Box No. III** | **Observations where unity of invention is lacking (Continuation of item 3 of first sheet)** |
|---|---|

This International Searching Authority found multiple inventions in this international application, as follows:
   (See extra sheet.)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.:
   Claim 1, claim 2 and all claims depending on claim 2.

| **Remark on Protest** | ☐ The additional search fees were accompanied by the applicant's protest and, where applicable, payment of a protest fee. |
|---|---|
| the | ☐ The additional search fees were accompanied by the applicant's protest but the applicable protest fee was not paid within the time limit specified in the invitation. |
| | ☐ No protest accompanied the payment of additional search fees. |

Form PCT/ISA/210 (continuation of first sheet (2)) (April 2007)

**INTERNATIONAL SEARCH REPORT**

International application No.

PCT/JP2008/064691

The matter common to the inventions according to claims 1 to 7 resides in the matter as set forth in claim 1.

As the results of the search, however, it has been clarified that the above-described matter is not novel because of having been disclosed in documents JP 11-216163 A (Oji PAPER Co., Ltd.), August 10, 1999 (08.10.99), claim 1, paragraph [0009], Figs. 1 to 3; and JP 2003-153939 A (LIVEDO Corp.), May 27, 2003 (05.27.03), paragraphs [0010] and [0016], etc.

As a result, the matter as described above falls within the category of prior art and, therefore, this common matter cannot be considered as a special technical feature.

Such being the case, there is no matter common to all of the inventions according to claims 1 to 7.

Considering the number of invention groups that are so linked as to form a single general inventive concept as described in the present invention, i.e., the number of invention groups, it is recognized based on the dependency of claims of the present application that there are at least the following three series.

Claim 1, claim 2 depending on claim 1, claims 3 and 6 depending on claim 2 (the rest is omitted).

Claim 1, claim 3 depending on claim 1, claims 4 to 6 depending on claim 3 (the rest is omitted).

Claim 1, claim 6 depending on claim 1 and claims 7 depending on claim 6.

Since there is no "special technical matter" common to the above three series in the present invention, it is recognized that the present invention has at least three groups of inventions.

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007097979 A